# FASCICULE DE BREVET EUROPEEN

(11) **EP 1 427 345 B1**
(45) Date de publication et mention de la délivrance du brevet: **09.02.2005**
(21) Numéro de dépôt: 02783170.0
(22) Date de dépôt: 17.09.2002
(51) Int. Cl.: A61B 19/00

(54) **DISPOSITIF DE CONTENTION NON INVASIF POUR LE TRAITEMENT EN RADIOTHERAPIE**
NICHTINVASIVE HALTEVORRICHTUNG FÜR DIE STRAHLENTHERAPIE
NON-INVASIVE RETENTION DEVICE FOR RADIOTHERAPY TREATMENT

(30) Priorité: 17.09.2001 FR 0112013
(43) Date de publication de la demande: 16.06.2004
(73) Titulaire: CENTRE HOSPITALIER REGIONAL ET UNIVERSITAIRE DE LILLE, 59037 Lille Cédex (FR); Centre Oscar Lambret, 59020 Lille Cedex (FR); UNIVERSITE DU DROIT ET DE LA SANTE DE LILLE, 59800 Lille (FR); UNIVERSITE DES SCIENCES ET TECHNOLOGIES DE LILLE, 59655 Villeneuve-d'Ascq Cedex (FR)
(72) Inventeur: BOUREL, Philippe, F-59242 Genech (FR); CAUDRELIER, Jean, Michel, F-59000 Lille (FR); GIBON, David, F-59000 Lille (FR); ROUSSEAU, Jean, F-37000 Tours (FR)
(74) Mandataire: Hennion, Jean-Claude
(86) Numéro de dépôt international: PCT/FR2002/003169
(87) Numéro de publication internationale: WO 2003/024351

(56) Documents cités:
- WO-A-98/41282
- WO-A-98/55018
- DE-A- 19 626 910

## Description

La présente invention concerne un dispositif de contention non invasif pour le traitement en radiothérapie qui peut également être utilisé en imagerie médicale pour la localisation d'une lésion à traiter ou d'organes sains à éviter.

La radiothérapie est utilisée aussi bien pour le traitement de tumeurs cancéreuses que pour le traitement de lésions non tumorales et non cancéreuses de type, par exemple, malformations artério-veineuses. Le traitement de radiothérapie consiste à délivrer en une ou plusieurs séances une dose d'irradiation sur la lésion à traiter encore appelée cible par un ou plusieurs faisceaux d'irradiation. Il est donc important que le patient conserve toujours, d'une séance à l'autre, la même position déterminée en sorte que l'irradiation atteigne effectivement la cible en épargnant au maximum les tissus sains avoisinants. Pour ce faire, des systèmes de contention ont été développés afin de garantir la reproductibilité du positionnement du patient lors des différentes séances de radiothérapie et ceci pour chaque faisceau d'irradiation.

Il existe deux types de système de contention : les systèmes de contention invasifs et les systèmes de contention non invasifs.

Les systèmes de contention invasifs comprennent des éléments de contention qui viennent coopérer avec des vis ou autres types de fixations implantées directement dans la table osseuse du crâne du patient. Ils sont généralement utilisés dans le cas d'irradiations à dose élevée en séance unique dans le cadre d'un traitement communément appelé radiochirurgie stéréotaxique.

Les systèmes de contention non invasifs sont des systèmes externes qui viennent se positionner sur la tête du patient sans prise directe sur sa boite crânienne. L'invention concerne ce deuxième type de système de contention.

Les lésions à traiter sont détectées par une ou plusieurs techniques d'imagerie médicale, telle que par exemple, l'imagerie tomographique numérique (TDM), l'Imagerie par Résonance Magnétique (IRM) ou la Tomographie par Emissions de Positrons (TEP) qui permettent de reconstruire et de visualiser les structures cérébrales du patient en trois dimensions.

La lésion détectée en imagerie est tout d'abord localisée spatialement par rapport à un référentiel lié au dispositif d'imagerie (référentiel imagerie). L'appareil de radiothérapie possède lui-même son propre référentiel (référentiel traitement) dont le centre se situe à l'isocentre de l'appareil, qui correspond au point de concordance des différents faisceaux d'irradiation. L'isocentre de l'appareil est, en général, matérialisé par l'intersection de faisceaux lumineux émis par des positionneurs lasers fixés aux murs (2 latéraux et 1 longitudinal).

Le traitement de radiothérapie est mis en oeuvre en alignant le centre de la cible détectée en imagerie avec l'isocentre de l'appareil de radiothérapie, ce qui implique un changement de référentiel.

Pour effectuer ce changement de référentiel, la méthode la plus habituelle consiste à utiliser un référentiel lié au patient, qui permet le passage de la position de la cible dans le référentiel imagerie à sa position dans le référentiel de traitement. Cette méthode consiste à matérialiser le référentiel patient en identifiant, sur la peau du patient ou sur le système de contention, les points issus de la projection des faisceaux lumineux émis par les lasers qui servent à matérialiser l'isocentre. Sur ces points, il est possible de positionner des marqueurs visibles en imagerie dont les positions peuvent être connues dans le référentiel imagerie. La connaissance de la position de la cible par rapport aux marqueurs permet de connaître la position de la cible dans le référentiel de traitement.

Le positionnement de cette cible à l'isocentre de l'appareil de traitement est effectué en déplaçant le référentiel patient - et donc le patient lui-même- pour amener la cible à l'isocentre.

La méthode utilisant la matérialisation des points sur la peau du patient ne permet pas d'assurer la contention du patient durant le traitement. Les systèmes de contention non invasifs connus sont souvent inadaptés pour une utilisation à la fois en imagerie médicale et en radiothérapie.

Ainsi, le dispositif décrit dans le document FR 2 571 605 permet effectivement une contention non invasive particulièrement supportable car le patient est maintenu au niveau des oreilles et du nez sur un socle équipé d'un coussin et il semble adaptable à de nombreuses morphologies. Il permet un contrôle du repositionnement par l'utilisation de graduations et un repérage en imagerie de la lésion du fait de la visualisation de deux structures triangulaires disposées latéralement sur la tête du patient. En revanche, la disposition des barres de repérage impose des contraintes de positionnement en imagerie et l'encombrement global du système ne permet pas son utilisation optimale dans tous les dispositifs d'imagerie. De la même façon, ce dispositif qui est conçu en sorte de permettre le traitement chirurgical de la lésion s'avère mal adapté pour être utilisé en radiothérapie. En effet, le référentiel patient défini par ce dispositif n'est pas un référentiel orthonormé centré comme celui de l'appareil de radiothérapie. En conséquence, il est très difficile de passer du référentiel patient à celui de l'appareil de radiothérapie sans l'utilisation d'un système informatique dédié spécialement à cette tâche. Il est également très difficile de faire coïncider ces référentiels par la technique des lasers précédemment décrite du fait de la configuration du dispositif et des matériaux utilisés. En outre, les matériaux utilisés (plastique et métal) absorbent le rayonnement, et perturbent la dose délivrée à la cible. Enfin, dans le dispositif décrit, les moyens de contention au niveau des oreilles sont fixés sur les barres de repérage et le réglage du dispositif sur la tête du patient est obtenu en déformant un arceau de contention qui porte les moyens de contention au niveau du nez, et en modifiant de manière préjudiciable la position dans l'espace des barres de repérage.

D'autres dispositifs, comme par exemple, celui décrit dans le document US 5 531 229 maintiennent le patient dans une position déterminée au moyen notamment d'un élément de positionnement buccal que le patient maintient serré entre ses dents. Si la reproductibilité du positionnement est bonne du fait de la fabrication d'éléments de positionnement buccaux personnalisés, la contention est difficilement supportable et ne permet pas au patient de communiquer. Le positionnement lors du traitement de radiothérapie implique l'ajout de plaques au niveau des oreilles du patient, les faisceaux laser qui matérialisent le référentiel de l'appareil de traitement venant frapper ces plaques ce qui permet de positionner la lésion préalablement détectée dans ce référentiel. Ce dispositif est trop encombrant pour pouvoir être utilisé dans certains appareils d'imagerie comme par exemple dans l'antenne tête d'un appareil IRM, cette antenne étant particulièrement étroite afin d'augmenter la précision de l'imagerie et donc du repérage.

On a également déjà proposé dans la demande de brevet WO 98/55018 un dispositif de stéréotaxie prévu pour être utilisé dans différents types d'imagerie, qui correspond généralement au préambule de la revendication 1. Cependant, ce dispositif est utilisable uniquement en imagerie médicale, et n'est pas un dispositif de contention utilisable en radiothérapie ; notamment, il ne permet pas d'immobiliser dans une position prédéfinie et reproductible la tête d'un patient sur la table d'un appareil de radiothérapie.

Le but de la présente invention est donc de proposer un dispositif de contention non invasif qui est utilisable à la fois pour le traitement en radiothérapie et pour le repérage en imagerie médicale et qui ne présente pas les inconvénients du dispositif décrit dans la demande de brevet français précitée FR 2 751 605.

Ce but est atteint au moyen du dispositif de contention de la revendication 1.

Selon l'invention, le premier arceau, qui sert de support aux premiers et seconds moyens de localisation repérables respectivement en imagerie et en radiothérapie, étant rigide, il n'y aucune déformation possible de cet arceau qui conduirait à un changement de position de ces moyens de localisation par rapport à la tête du patient, entre deux séances de traitement ou entre le repérage en imagerie et le traitement en radiothérapie. De plus, les premiers et les seconds moyens de repérage définissant deux référentiels qui possèdent le même centre qui correspond au centre du référentiel lié au patient, il est ainsi très facile, sans logiciel spécifiquement dédié à cette tâche de passer du référentiel utilisé en imagerie (et qui dépend donc des premiers moyens de localisation) à celui utilisé en radiothérapie (et qui dépend donc des seconds moyens de localisation), la position relative des premiers moyens de localisation et des seconds moyens de localisation étant parfaitement connue et déterminée.

L'encombrement du dispositif de l'invention est très faible du fait de l'utilisation de moyens de contention et de repérage particuliers, ce qui permet son utilisation dans tous les appareils d'imagerie existants et même dans l'antenne tête d'un appareil d'IRM.

Par ailleurs, les premiers moyens de localisation étant disposés au niveau du premier arceau, ils sont donc proches du crâne du patient ce qui permet l'utilisation en imagerie de champs de vue réduits afin de limiter les déformations géométriques éventuelles et d'utiliser des images de meilleure définition.

La contention du patient sur la table de radiothérapie ou dans l'appareil d'imagerie si nécessaire peut être assurée de toute manière connue. Selon un mode de réalisation particulier, le dispositif de contention de l'invention comporte un socle apte à être fixé sur la table d'un appareil médical et en particulier d'un appareil de radiothérapie et des moyens de fixation du premier arceau sur le socle. L'utilisation d'un tel socle permet d'assurer la contention du patient sur la table de traitement de radiothérapie et donc de garantir un positionnement fixe et reproductible du patient durant tout la durée du traitement.

La disposition et le mode de fixation des premiers et seconds moyens de localisation ne sont pas limitées selon la présente invention. Ainsi, les premiers moyens et/ou les seconds moyens de localisation peuvent être solidaires du premier arceau ou disposés sur un support amovible qui coopère avec ce premier arceau.

Ainsi, selon une variante de réalisation, le dispositif de l'invention comporte un second arceau qui coopère de manière amovible avec le premier arceau, ce second arceau comportant les éléments de repérage des premiers moyens de localisation et/ou des seconds moyens de localisation. Ce second arceau amovible permet de changer facilement les premiers moyens de localisation utilisables en imagerie ce qui permet d'utiliser le dispositif de l'invention dans différents appareils d'imagerie sans modifications de sa structure.

La présente invention, ses caractéristiques et les différents avantages qu'elle procure apparaîtront mieux à la lecture de la description qui suit et fait référence aux dessins annexés représentant un mode particulier de réalisation, présents à titre d'exemples non limitatif et sur lesquels :
- la figure 1 représente une vue en perspective d'un mode de réalisation particulier du dispositif de l'invention ; et
- la figure 2 représente le dispositif de la figure 1 mis en place sur la tête d'un patient.

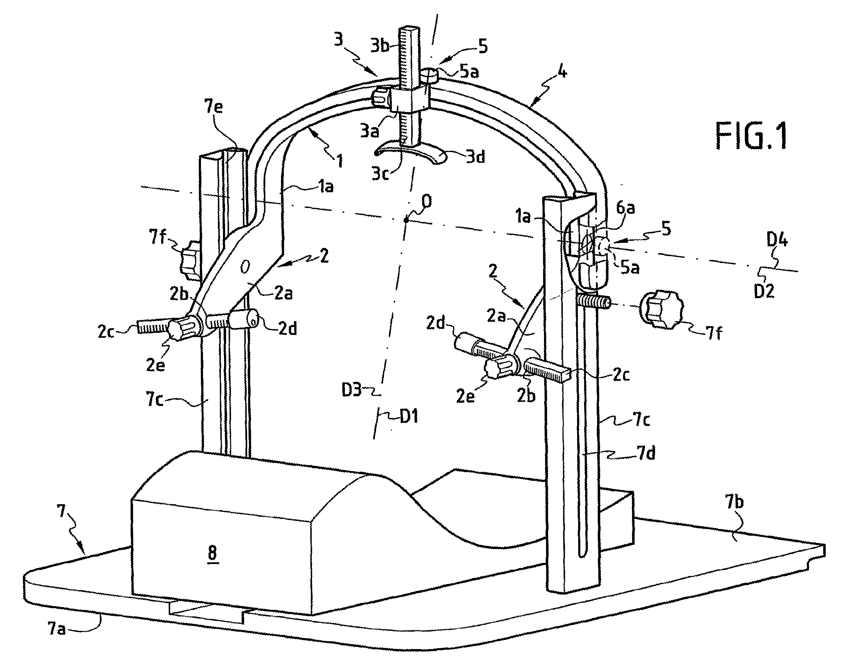

En référence à la figure 1, le dispositif de l'invention comporte un premier arceau 1 rigide dont les deux extrémités la sont équipées de premiers moyens de contention 2 de l'arceau 1 sur la tête du patient. Ces premiers moyens de contention 2 comportent une plaque 2a munie d'un trou 2b à travers lequel passe une tige creuse 2c ouverte à ses deux extrémités. La tige creuse 2c coopère avec le trou 2b en sorte de permettre le réglage de la longueur de la tige 2c dépassant vers l'intérieur du premier arceau 1. Les tiges creuses 2c sont munies à une de leurs extrémités d'un tampon perforé 2d. La tige creuse et plus particulièrement le tampon 2d est destiné à venir en butée contre la paroi interne du conduit auditifs du patient. Les tiges 2c étant creuses et ouvertes à leurs deux extrémités et les tampons 2d étant perforés, il n'y a donc aucune surpression au niveau du tympan du patient qui pourrait lui causer un quelconque inconfort. En outre, la capacité auditive du patient n'est pas trop altérée, lorsque l'arceau de contention 1 est positionné sur sa tête.

Les tiges 2c sont graduées afin de permettre le repositionnement précis et sont fixées à la plaque 2a à l'aide d'une vis 2e qui permet soit le coulissement de la tige 2c dans le trou 2b soit, par serrage, la fixation de la position de la tige 2c dans le trou 2b.

Dans une autre réalisation, chaque tige creuse pourrait également comporter un canal longitudinal interne débouchant uniquement au niveau de l'une de ses deux extrémités (extrémité destinée à être introduite dans le conduit auditif), ledit canal longitudinal communiquant par ailleurs avec l'extérieur via un ou plusieurs conduits débouchants, qui s'étendent transversalement au canal longitudinal et qui font office d'évents.

Des seconds moyens de contention 3 destinés à coopérer avec l'os du nez du patient sont disposés sensiblement dans l'axe de symétrie du premier arceau 1. Ces seconds moyens de contention comportent un support 3a solidaire du premier arceau 1, s'étendant dans un plan tangent à la partie supérieure du premier arceau et dans une direction sensiblement perpendiculaire à cette dernière et une tige 3b qui coopère avec le support 3a. L'extrémité 3c de la tige 3b est munie d'un support nasal 3d obtenu par moulage d'une empreinte personnalisée sur le nez du patient. Le support nasal 3d étant personnalisé en fonction de la morphologie du patient, il permet d'assurer un positionnement fiable du dispositif de l'invention sur le patient. La tige 3b est graduée afin de permettre le repositionnement précis et elle est solidarisée au support 3a à l'aide d'une vis 3e qui vient fixer en position la tige 3b.

Le mode de réalisation particulier représenté sur la figure 1 comporte également un second arceau 4 rigide et amovible qui coopère avec le premier arceau 1. Ce second arceau amovible 4 comporte des premiers moyens de localisation 5 utilisables en imagerie. Ces premiers moyens de localisation 5 comportent dans le cas particulier ici représenté trois éléments de repérage 5a ponctuels, réalisés dans un matériau adapté, par exemple, pour la localisation en TDM, du type liquide riche en protons pour l'IRM ou du type produit de contraste pour le TEP. Ces trois éléments ponctuels 5a sont disposés selon deux droites perpendiculaires D1 et D2 sécantes au point O qui correspond au centre du référentiel lié au patient (et donc au dispositif de contention de l'invention). Dans l'exemple particulier représenté, un élément 5a est disposé au milieu de l'arceau 4 tandis que les deux autres sont disposés respectivement au niveau de chacune des extrémités de cet arceau.

Dans le mode de réalisation particulier représenté, les deux arceaux 1 et 4 coopèrent par emboîtement. Le second arceau 4 comporte trois ergots (non représentés) qui viennent s'encliqueter dans trois ouvertures (non représentées) ménagées dans le premier arceau 1. Les deux arceaux 1 et 4 se superposent parfaitement l'un sur l'autre lorsqu'ils sont fixés l'un à l'autre. formant ainsi un ensemble rigide.

Le premier arceau 1 comporte également des seconds moyens de localisation 6 utilisables lors de la séance de radiothérapie. Dans l'exemple particulier représenté, ces seconds moyens de localisation 6 comportent trois croix 6a marquées sur la surface du premier arceau 1. Ces trois croix 6a sont disposées selon deux droites perpendiculaires D3 et D4 et sécantes au point O précité. Dans l'exemple particulier représenté, les droites D1 et D2 se confondent avec les droites D3 et D4. Ainsi, dans l'exemple représenté, une croix 6a est disposée au niveau du milieu de l'arceau 1 tandis que deux autres croix sont disposées respectivement au niveau de chacune des extrémités de ce dernier. La disposition des seconds moyens de localisation 6 n'est pas limitative de l'invention. Ainsi l'homme du métier est en mesure d'adapter cette disposition en fonction de la position des lasers dans la salle de radiothérapie. Notamment, il est possible d'équiper non par le premier arceau 1 lui-même mais par exemple la tige 3b d'une croix 6a ou de tout autre éléments de repérage utilisable lors du traitement de radiothérapie.

Le dispositif de l'invention comporte également un socle 7 dont la surface inférieure 7a est destinée à être fixée, par exemple au moyen de vis (non représentés) ou posée sur la table de radiothérapie. La seconde face 7b est équipée d'un coussin 8 dont la forme est adaptée pour caler la nuque du patient. Deux montants 7c parallèles prolongent le socle 7 dans un plan perpendiculaire à la seconde face 7b. Chacun des montant 7c est en fait un rail creux dont la face située en regard du coussin 8 est pourvue d'une lumière allongée 7d. Un guide 7e solidaire du cadre 1 coopère avec la lumière 7d. La plaque 1a comporte une légère protubérance 1c qui vient s'encastrer dans le rail formé par le montant 7c et qui permet de bloquer l'arceau 1 en rotation tout en permettant son guidage par translation le long de la lumière 7d. Le réglage de la position du premier arceau est mis en oeuvre par coulissement du guide 7e dans la lumière 7d. Le blocage en position de l'arceau 1 s'effectue à l'aide de la molette 7f qui se visse sur le guide 7e et vient en butée contre le montant 7c au niveau des bords de la lumière 7d maintenant ainsi le premier arceau 1 en position. La présence des montants 7c permet d'une part d'assurer une contention efficace du patient sur la table de traitement tout en évitant de former écran aux radiations. La coopération du premier arceau 1 avec les montants 7c permet de tenir compte de l'anatomie du patient en autorisant le réglage de la position en hauteur du premier arceau 1 par rapport au socle 7.

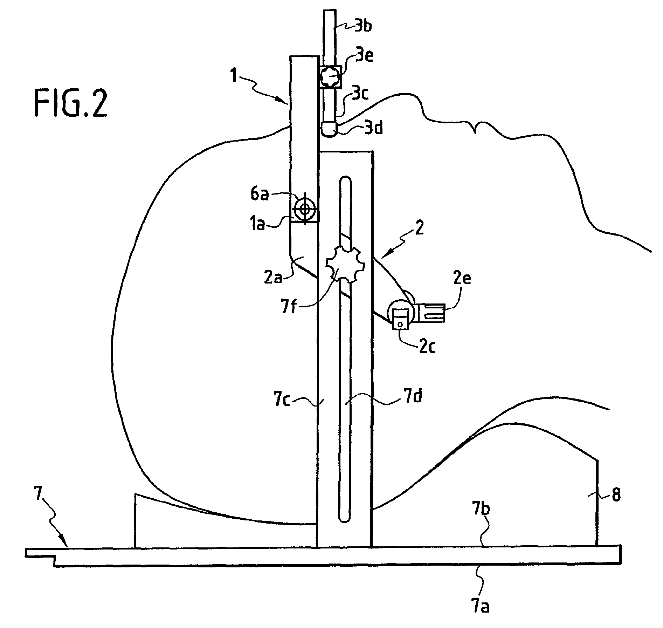

En référence à la figure 2, lorsque le dispositif de l'invention est fixé sur le patient, les tiges creuses 2c coopèrent avec les conduits auditifs de ce dernier tandis que le support nasal 3d coopère avec son nez. Ces deux moyens de contention assurent la fixation du premier arceau 1 sur la tête du patient sans déformation de l'arceau 1.

L'utilisation du dispositif de la présente invention va maintenant être plus amplement expliqué.

Pour le repérage en imagerie médicale de la lésion, le patient muni du premier arceau 1 sur lequel est fixé le second arceau 4 est introduit, par exemple dans l'antenne tête d'un dispositif IRM. Le second arceau amovible 4 équipé d'élément de repérage adaptés au dispositif d'imagerie utilisé est au préalable fixé sur le premier arceau 1. Selon le type de dispositif d'imagerie utilisé, il est également possible d'utiliser les deux arceaux 1 et 4 fixé sur le socle 7. Dans un dispositif du type scanner, par exemple, il est possible d'utiliser le socle 7 précité. La lésion est repérée en trois dimensions et ses coordonnées peuvent être calculées dans le référentiel lié au dispositif de l'invention, c'est-à-dire le référentiel (O, D1, D2). Les graduations des tiges 2c et 3b sont notées.

Pour le traitement de radiothérapie, le patient est allongé sur la table de radiothérapie, la tête sur le coussin 8. Le premier arceau 1 est laissé libre de coulisser le long des montants 7c. Le second arceau 4 peut être retiré à tout moment. Dans un premier temps, on repositionne exactement le premier arceau 1 sur le patient en positionnant les tiges creuses 2c et la tige 3b aux mêmes graduations que précédemment notées. La position de ces éléments doit être exactement la même que celles qu'ils occupaient durant l'étape de repérage en imagerie médicale. Le premier arceau 1 est ensuite solidarisé sur les montants 7c du socle 7 à l'aide de chacune des molettes 7f précitées. On déplace alors la table de. traitement avec le patient en sorte que les trois faisceaux lasers de positionnement qui équipent la salle de radiothérapie viennent frapper chacune des trois croix 6a. Lorsque cette opération est effectuée, on sait que le point qui correspond à l'intersection des trois faisceaux lasers et qui correspond donc à l'isocentre de l'appareil de radiothérapie coïncide avec le centre 0 du référentiel (O, D3, D4). Connaissant les coordonnées de la lésion dans le référentiel (O, D1, D2) et pouvant sans logiciel de calcul spécifique calculer simplement les coordonnées de la lésion dans le référentiel (O, D3, D4), il est aisé de déplacer le patient en sorte que le point d'intersection des trois faisceaux lasers coïncident avec la lésion à traiter dans le référentiel (O, D3, D4). La hauteur du premier arceau, qui est notée sur les montants 7c du socle 7, est notée et permet un repositionnement rapide lors des différentes séances. Dans le cas présent, les droites D1 et D2 étant confondues respectivement avec les droites D3 et D4, les coordonnées de la lésion sont les mêmes dans les deux référentiels.

Dans le cas où les précitées droites ne sont pas confondues, le changement de référentiel est aisé car il dépend uniquement de la position relative des éléments de repérage 5a et 6a qui étant fixés sur des arceaux rigide n'est pas susceptible d'être modifiée, notamment par exemple, par déformation mécanique du dispositif pendant sont utilisation.

Il est également possible selon l'invention d'équiper le premier arceau 1 de moyens de localisation repérables en imagerie, les seconds moyens de localisation utilisables en radiothérapie pouvant être disposés soit sur ce même premier arceau soit sur un second arceau amovible.

## Revendications

1. Dispositif de contention non invasif pour le traitement en radiothérapie et utilisable en imagerie médicale notamment pour la localisation des lésions cérébrales, ledit dispositif comporte :
- un premier arceau (1) ;
- des premiers moyens de contention (2) dudit premier arceau (1) sur le patient qui sont aptes à être positionnés au niveau de chacune des oreilles dudit patient ;
- des deuxièmes moyens de contention (3) du premier arceau sur le patient, aptes à être positionnés au niveau du nez du patient ;
- des premiers moyens de localisation (5) utilisables en imagerie médicale ;
- des moyens (7, 7c) de solidarisation du premier arceau sur une table d'un appareil médical ;
ledit dispositif est **caractérisé en ce qu'**il comporte des seconds moyens de localisation (6) repérables lors de la radiothérapie, **en ce que** le premier arceau (1) est rigide et sert de support aux premiers (5) et seconds (6) moyens de localisation, lesdits premiers et seconds moyens (5 ; 6) de localisation comprennent chacun au moins trois éléments de repérage (5a ; 6a), lesdits éléments de repérage (5a) des premiers moyens de localisation définissant deux droites (D1 ; D2) perpendiculaires et sécantes en un point appelé centre (O), lesdits éléments de repérage (6a) desdits seconds moyens de localisation définissant également deux droites (D3 ; D4) perpendiculaires et sécantes audit point appelé centre (O), ledit point appelé centre (O) correspondant au centre du référentiel lié au patient.

2. Dispositif de contention selon la revendication 1, **caractérisé en ce que** les moyens de solidarisation comportent un socle (7), des moyens de fixation (7c) dudit premier arceau (1) sur ledit socle (7).

3. Dispositif selon la revendication 1 ou 2, **caractérisé en ce qu'**il comporte un deuxième arceau (4) qui coopère de manière amovible avec ledit premier arceau (1), ledit second arceau (4) comportant lesdits éléments de repérage (5a ; 6a) desdits premiers moyens de localisation et/ou desdits seconds moyens de localisation (5 ; 6).

4. Dispositif selon l'une quelconque des revendications 1 à 3, **caractérisé en ce que** lesdits éléments de repérage (6a) des seconds moyens de localisation sont du type croix.

5. Dispositif selon l'une quelconque des revendications 1 à 4, **caractérisé en ce que** les deux droites (D1 ; D2) définies par lesdits éléments de repérage (5a) desdits premiers moyens de localisation (5) se confondent avec les deux droites (D3 ; D4) définies par lesdits éléments de repérage (6a) desdits seconds moyens de localisation (6).

6. Dispositif selon l'une quelconque des revendications 1 à 5, **caractérisé en ce que** lesdits seconds moyens de contention (3) destinés à coopérer avec le nez du patient comprennent un support nasal (3d) obtenu par moulage d'une empreinte personnalisée sur le nez du patient.

7. Dispositif selon l'une quelconque des revendications 1 à 6, **caractérisé en ce que** lesdits premiers moyens de contention (2) comportent des tiges creuses (2c) aptes à être en partie enfoncées dans le conduit auditif du patient et ouvertes en sorte d'éviter une surpression au niveau du tympan.

8. Dispositif selon l'une quelconque des revendications 2 à 7, **caractérisé en ce que** lesdits moyens de fixation dudit arceau sur ledit socle comprennent deux montants (7c) latéraux qui coopèrent avec ledit premier arceau (1) en sorte de permettre le réglage en hauteur dudit premier arceau (1) par rapport audit socle (7).

9. Dispositif selon l'une des revendications 1 à 8 **caractérisé en ce que** les premiers (2) et deuxièmes (3) moyens de contention permettent d'adapter le premier arceau de contention (1) sur un patient, sans déformation dudit arceau.

## Claims

1. A non-invasive retention device for radiotherapy treatment which can be used in medical imaging, particularly for locating cerebral lesions, said device comprising:
- a first arch (1);
- first retention means (2) of said first arch (1) on the patient which are to be positioned at the level of each of the ears of said patient;
- second retention means (3) of the first arch on the patient, to be positioned at the level of the nose of the patient;
- first locating means (5) which can be used in medical imaging;
- means (7, 7c) for fixing the first arch on a table of medical equipment;
said device is **characterised in that** it comprises second locating means (6) which are locatable during radiotherapy, **in that** the first arch (1) is rigid and serves as support to the first (5) and second (6) locating means, said first and second locating means (5; 6) each comprise at least three locating elements (5a; 6a), said locating elements (5a) of the first locating means defining two perpendicular straight lines (D1; D2) and intersecting at a point called centre (O), said locating elements (6a) of said second locating means also defining two perpendicular straight lines (D3; D4) and intersecting with said point known as centre (O), said point known as centre (O) corresponding to the centre of the reference frame attached to the patient.

2. A retention device according to Claim 1, **characterised in that** the fixing means comprise a pedestal (7), fixing means (7c) of said first arch (1) on said pedestal (7).

3. The device according to Claim 1 or 2, **characterised in that** it comprises a second arch (4) which cooperates in a removable way with said first arch (1), said second arch (4) comprising said locating elements (5a; 6a) of said first locating means and/or of said second locating means (5; 6).

4. The device according to any one of Claims 1 to 3, **characterised in that** said locating elements (6a) of the second locating means are of the cross type.

5. The device according to any one of Claims 1 to 4, **characterised in that** the two straight lines (D1; D2) defined by said locating elements (5a) of said first locating means (5) are merged with the two straight lines (D3; D4) defined by said locating elements (6a) of said second locating means (6).

6. The device according to any one of Claims 1 to 5, **characterised in that** said second retention means (3) for cooperating with the nose of the patient comprise a nasal support (3d) obtained by moulding a customised imprint on the nose of the patient.

7. The device according to any one of Claims 1 to 6, **characterised in that** said first retention means (2) comprise hollow rods (2c) to be in part driven into in the auditory canal of the patient and open so as to avoid excess pressure on the eardrum.

8. The device according to any one of Claims 2 to 7, **characterised in that** said fixing means of said arch on said pedestal comprise two lateral posts (7c) which cooperate with said first arch (1) so as to allow height adjustment of said first arch (1) relative to said pedestal (7).

9. The device according to one of Claims 1 to 8, **characterised in that** the first (2) and second (3) retention means allow the first retention arch (1) to be adapted to a patient, without deformation of said arch.

## Patentansprüche

1. Nicht-invasive Haltevorrichtung für die Strahlentherapiebehandlung und einsetzbar in der medizinischen Bildgebung, insbesondere zur Lokalisierung von Gehirnläsionen, die folgendes umfaßt:
- einen ersten Bogen (1);
- erste Mittel (2) zum Halten des ersten Bogens (1) an dem Patienten, die geeignet sind, im Bereich eines jeden Ohrs des Patienten positioniert zu werden;
- zweite Mittel (3) zum Halten des ersten Bogens an dem Patienten, welche geeignet sind, im Bereich der Nase des Patienten positioniert zu werden;
- erste Lokalisierungsmittel (5), die in der medizinischen Bildgebung einsetzbar sind;
- Mittel (7, 7c) zum festen Verbinden des ersten Bogens mit einem Tisch eines medizinischen Gerätes;
wobei die Vorrichtung **dadurch gekennzeichnet ist, daß** sie zweite Lokalisierungsmittel (6) enthält, die während der Strahlentherapie erfaßbar sind, daß der erste Bogen (1) starr ist und als Träger für die ersten (5) und zweiten (6) Lokalisierungsmittel dient, die ersten und zweiten Lokalisierungsmittel (5; 6) jeweils wenigstens drei Erfassungselemente (5a; 6a) enthalten, wobei die Erfassungselemente (5a) der ersten Lokalisierungsmittel zwei Geraden (D1; D2) definieren, die senkrecht verlaufen und sich in einem sogenannten Zentrumspunkt (O) schneiden, wobei die Erfassungselemente (6a) der zweiten Lokalisierungsmittel ebenfalls zwei Geraden (D3; D4) definieren, die senkrecht verlaufen und sich in dem sogenannten Zentrumspunkt (O) schneiden, wobei der sogenannte Zentrumspunkt (O) dem Mittelpunkt des mit dem Patienten verbundenen Bezugssystems entspricht.

2. Haltevorrichtung nach Anspruch 1, **dadurch gekennzeichnet, daß** die Mittel zum festen Verbinden einen Sockel (7), Mittel (7c) zum Befestigen des ersten Bogens (1) an dem Sockel (7) umfassen.

3. Vorrichtung nach Anspruch 1 oder 2, **dadurch gekennzeichnet, daß** sie einen zweiten Bogen (4) enthält, der mit dem ersten Bogen (1) lösbar zusammenwirkt, wobei der zweite Bogen (4) die genannten Erfassungselemente (5a; 6a) der ersten Lokalisierungsmittel und/oder der zweiten Lokalisierungsmittel (5; 6) umfaßt.

4. Vorrichtung nach irgendeinem der Ansprüche 1 bis 3, **dadurch gekennzeichnet, daß** die Erfassungselemente (6a) der zweiten Lokalisierungsmittel von der Art Kreuz sind.

5. Vorrichtung nach irgendeinem der Ansprüche 1 bis 4, **dadurch gekennzeichnet, daß** die zwei Geraden (D1; D2), welche durch die Erfassungselemente (5a) der ersten Lokalisierungsmittel (5) definiert werden, mit den zwei Geraden (D3; D4), die durch die Erfassungselemente (6a) der zweiten Lokalisierungsmittel (6) definiert werden, verschmelzen.

6. Vorrichtung nach irgendeinem der Ansprüche 1 bis 5, **dadurch gekennzeichnet, daß** die zweiten Haltemittel (3), welche dazu bestimmt sind, mit der Nase des Patienten zusammenzuwirken, eine Nasenauflage (3d) aufweisen, welche durch Formen eines personalisierten Abdrucks auf der Nase des Patienten erhalten wird.

7. Vorrichtung nach irgendeinem der Ansprüche 1 bis 6, **dadurch gekennzeichnet, daß** die ersten Haltemittel (2) Hohlstifte (2c) aufweisen, die geeignet sind, teilweise in den Gehörgang des Patienten hineingesteckt zu werden und die offen sind, um einen Überdruck im Bereich des Trommelfells zu vermeiden.

8. Vorrichtung nach irgendeinem der Ansprüche 2 bis 7, **dadurch gekennzeichnet, daß** die Mittel zum Befestigen des Bogens an dem Sockel zwei Seitenpfosten (7c) aufweisen, die mit dem ersten Bogen (1) zusammenwirken, um die Höhenverstellung des ersten Bogens (1) gegenüber dem Sockel (7) zu ermöglichen.

9. Vorrichtung nach einem der Ansprüche 1 bis 8, **dadurch gekennzeichnet, daß** die ersten (2) und zweiten (3) Haltemittel ermöglichen, den ersten Haltebogen (1) an einen Patienten anzupassen, ohne den Bogen zu verformen.
